(19) **Europäisches Patentamt European Patent Office Office européen des brevets**

(11) **EP 3 970 794 A1**

(12) **EUROPEAN PATENT APPLICATION**
published in accordance with Art. 153(4) EPC

(43) Date of publication:
**23.03.2022 Bulletin 2022/12**

(21) Application number: **20805906.3**

(22) Date of filing: **14.05.2020**

(51) International Patent Classification (IPC):
*A61P 25/00* [(2006.01)]   *C12N 5/079* [(2010.01)]
*C12N 5/0797* [(2010.01)]   *A61K 35/30* [(2015.01)]
*A61K 35/38* [(2015.01)]   *A61L 27/38* [(2006.01)]

(52) Cooperative Patent Classification (CPC):
**A61K 35/30; A61K 35/38; A61L 27/38; A61P 25/00**

(86) International application number:
**PCT/JP2020/019290**

(87) International publication number:
**WO 2020/230856 (19.11.2020 Gazette 2020/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.05.2019 JP 2019091625**

(71) Applicant: **University of Tsukuba**
**Ibaraki 305-8577 (JP)**

(72) Inventors:
  • **MARUSHIMA Aiki**
    **Tsukuba-shi, Ibaraki 305-8577 (JP)**
  • **MATSUMURA Akira**
    **Tsukuba-shi, Ibaraki 305-8577 (JP)**

  • **BUKAWA Hiroki**
    **Tsukuba-shi, Ibaraki 305-8577 (JP)**
  • **KODA Masao**
    **Tsukuba-shi, Ibaraki 305-8577 (JP)**
  • **ISHIKAWA Hiroshi**
    **Tsukuba-shi, Ibaraki 305-8577 (JP)**
  • **OHYAMA Akihiro**
    **Tsukuba-shi, Ibaraki 305-8577 (JP)**
  • **TOYOMURA Junko**
    **Tsukuba-shi, Ibaraki 305-8577 (JP)**
  • **MATSUMARU Yuji**
    **Tsukuba-shi, Ibaraki 305-8577 (JP)**
  • **MATSUMURA Hideaki**
    **Tsukuba-shi, Ibaraki 305-8577 (JP)**
  • **WATANABE Miho**
    **Niigata-shi, Niigata 951-8151 (JP)**

(74) Representative: **Regimbeau**
    **20, rue de Chazelles**
    **75847 Paris Cedex 17 (FR)**

(54) **NERVOUS SYSTEM CELL POPULATION, NERVOUS SYSTEM CELL-CONTAINING PREPARATION AND METHOD FOR PRODUCING SAID POPULATION AND PREPARATION**

(57)    The present disclosure relates to a neural cell population, a neural cell-containing preparation, and a method for producing the population and preparation.

More particularly, the present invention relates to a neural cell population derived from intraoral mesenchymal cells, wherein a proportion of normal diploid cells is 80% or more, a preparation containing the neural cell population, and a method for producing the population and the preparation.

SCALE: 100 μm

FIG. 2

\201@\201@\201@\201@\201@\201@ SEQUENCE LISTING

<110> University of Tsukuba

<120>\201@Neural cell population,\201@neural cells-containing- formulation, and production method thereof

<130> 232202PX

<141> 2020-05-14

<150> JP 2019-091625

<151> 2019-05-14

<160> 6\201@

<170> PatentIn version 3.5

<210> 1
<211> 20
<212> DNA
<213> Artificial Sequence

<220>
<223> primer

<400> 1
accattgcat
cttggctgtc\201@\201@\201@\201@\201@\201@\201@\201@\201@\201@\201@\201@\201@20

<210> 2
<211> 20
<212> DNA
<213> Artificial Sequence

<220>
<223> primer

<400> 2
cgatgccctg
ctttaccaaa\201@\201@\201@\201@\201@\201@\201@\201@\201@\201@\201@\201@\201@20

<210> 3
<211> 20
<212> DNA
<213> Artificial Sequence

<220>
<223> primer

<400> 3
aaccccaaga
tgcacaactc\201@\201@\201@\201@\201@\201@\201@\201@\201@\201@\201@\201@\201@20

<210> 4
<211> 20
<212> DNA
<213> Artificial Sequence

```
<220>
<223> primer

<400> 4
cggggccggt
atttataatc\201@\201@\201@\201@\201@\201@\201@\201@\201@\201@\201@\201@\201@20


<210> 5
<211> 20
<212> DNA
<213> Artificial Sequence

<220>
<223> primer

<400> 5
aacagcgacg
gaggtctcta\201@\201@\201@\201@\201@\201@\201@\201@\201@\201@\201@\201@\201@20


<210> 6
<211> 20
<212> DNA
<213> Artificial Sequence

<220>
<223> primer

<400> 6
ttctcttgtc
ccgcagactt\201@\201@\201@\201@\201@\201@\201@\201@\201@\201@\201@\201@\201@20
```

**Description**

CROSS-REFERENCE TO RELATED APPLICATION

This Patent application claims priority to JP 2019-91625 A, filed on May 14, 2019, the entire disclosure of which is incorporated herein by reference.

BACKGROUND OF THE INVENTION

FIELD OF THE INVENTION

The present disclosure relates to a neural cell population, a neural cell-containing preparation, and a method for producing the population and preparation.

BACKGROUND ART

As for central nervous system injuries, in particular spinal cord injuries, transplantation of neural cells is required within 2 weeks from the injuries (Non-Patent Literature 1). Meanwhile, although there are many reports regarding induction of differentiation of mesenchymal stem cells into neural cells (Non-Patent Literatures 2 to 4), isolation of cells obtained by inducing neural differentiation of ectodermal mesenchymal tissue for several days has not been reported.
In order to obtain neural stem cells from tissue collected from the patient himself or herself, neural stem cells emerging using a neuron differentiation medium have to be separated using methods such as colonial cloning or cell sorter (Non-Patent Literature 5 and
Patent Document 1). However, colonial cloning is time-consuming, and cell sorter requires expensive devices and antibodies, and further, a burdensome operation such as cleaning of antibodies is unavoidable. It is also problematic that heavy damage is caused to neural stem cells during separation. In particular, in a case of autologous transplantation in which a cell-containing preparation and a material for regenerative medicine produced by processing cells collected from a patient himself or herself are transplanted to the patient, when it takes time to induce differentiation of cells collected from the patient into neural cells, there is a problem that autologous transplantation cannot be used for treatment in the acute or sub-acute phase.

PRIOR ART DOCUMENTS

PATENT DOCUMENTS

Patent Document 1: JP 2010-060576 A

NON-PATENT DOCUMENTS

Non-Patent Literature 1: Okano H, Okada S, Nakamura M, Toyama Y. Neural stem cells and regeneration of injured spinal cord. Kidney International, 68 (2005), 1927-31.
Non-Patent Literature 2: Kaukua N, Shahidi MK, Konstantinidou C, Dyachuk, V, Kaucka M, Furlan A, An Z, Wang L, Hultman I, Ahrlund-Rivhter L, Blom H, Brismar H, Lopes NA, Pachnis V, Suter U, Clevers H, Thesleff I, Sharpe P, Ernfors P, Fried K, Adameyko I. Glial origin of mesenchymal stem cells in a tooth model system. Nature 2014 Sep 25; 513(7519): 551-4.doi: 10.1038/nature13536.
Non-Patent Literature 3: Jang S, Jeong HS. Histone deacetylase inhibition-mediated neuronal differentiation via the Wnt signaling pathway in human adipose tissue derived mesenchymal stem cells.
Non-Patent Literature 4: Dong F, Fend E, Zheng T, Tian Y. In situ synthesized silver nanoclusters for tracking the role of telomerase activity in the differentiation of mesenchymal stem cells top neural stem cells. ACS Appl Mate Interfaces 2018 Jan 17; 10(2):2051-2057.
Non-Patent Literature 5: Li Xiao, Ryoji Ide, Chikako Saiki, Yasuo Kumazawa, Hisashi Okamura. Human Dental Pulp Cells Differentiate toward Neuronal Cells and Promote Neuroregeneration in Adult Organotypic Hippocampal Slices In Vitro. Int. J. Mol. Sci. 18, 1745 (2017).

SUMMARY OF THE INVENTION

As a result of diligent research, the inventors of the present disclosure have now found that cells obtained by initial culture of tissue collected from intraoral mesenchyme are cultured in a neuron differentiation medium, thereby obtaining, from the medium, a cell population containing high levels of neural cells, including neural stem cells. The present disclosure is based on these findings.

Accordingly, the present disclosure provides a method for rapidly producing a cell-containing preparation containing high levels of neural cells.

According to one embodiment of the present disclosure, there is provided a method for producing a neural cell-containing preparation, the method including:

preparing an adherent cell culture container in which initially cultured cells derived from intraoral mesenchymal tissue are cultured in a neuron differentiation medium; and

collecting cells that do not adhere to the adherent cell culture container from the neuron differentiation medium to obtain a cell population,

wherein the neural cell-containing preparation includes the cell population, or spheroids or neural cells derived from the cell population.

In addition, according to the other embodiment of the present disclosure, a neural cell preparation obtained by the above method is provided.

Also, according to the other embodiment of the present disclosure, there is provided a neural cell population derived from intraoral mesenchymal tissue cells, wherein a proportion of normal diploid cells is 80% or more.

According to the present disclosure, it is possible to rapidly produce a cell preparation containing high levels of neural cells. In addition, according to the present disclosure, it is also possible to easily produce a neural cell-containing preparation without an expensive device or an antibody. In particular, according to the present disclosure, even in a case of autologous transplantation in which a cell-containing preparation produced by processing cells collected from a patient himself or herself are transplanted to the patient, since differentiation of cells collected from the patient into neural cells can be induced in a short time, it is possible to produce a cell-containing preparation and a material for regenerative medicine for treatment by autologous transplantation in the acute or sub-acute phase. In addition, according to the method of the present disclosure, it is possible to provide a neural cell population in which the occurrence of chromosomal abnormalities is suppressed in differentiation induction and the proportion of normal diploid cells is high. Accordingly, the method of the present disclosure is very advantageous in providing a high-quality material for regenerative medicine.

BRIEF DESCRIPTION OF THE DRAWINGS

FIG. 1 is a photograph of cells obtained by dispersing human dental pulp tissue in a digestive enzyme solution and then performing primary culture. The scale in FIG. 1 is 100 $\mu$m.

FIG. 2 is a photograph of small free cells that appeared when initially cultured cells from the human dental pulp tissue were cultured in a neuron differentiation medium after adhered to a culture dish. The small free cells are observed as indicated by the leftward arrow. The scale in FIG. 2 is 100 $\mu$m.

FIG. 3 is a photograph captured immediately after seeding the small free cells collected by centrifugation on a non-adherent culture dish containing a neural maintenance medium. The scale in FIG. 3 is 100 $\mu$m.

FIG. 4 is a photograph captured when 24 hours have elapsed after seeding of the small free cells in the non-adherent culture dish containing a neural maintenance medium. Formation of spheroids is observed. The scale in FIG. 4 is 100 $\mu$m.

FIG. 5 is a photograph captured after 24 hours of culture of the spheroids in an adherent culture dish containing a neural maintenance medium. An outgrowth of neuron-like cells from the spheroids is observed. The scale in FIG. 5 is 100 $\mu$m.

FIG. 6 is a photograph captured after 10 days of culture of spheroids in an adherent culture dish containing a neural maintenance medium. A significant outgrowth of neuron-like cells from the spheroids is observed. The scale in FIG. 6 is 200 $\mu$m.

FIG. 7 include photographs of a specimen obtained by cutting a spheroid into a 5 $\mu$m section, and immunostaining the section with an anti-$\beta$III-tubulin antibody and DAPI. The photograph on the left side shows nuclei stained with DAPI. The photograph in the center shows expression of $\beta$III-tubulin. The photograph on the right side is obtained by superimposing the photograph on the left side and the photograph in the center. Most of the spheroid cells are positive for staining with the anti-$\beta$III-tubulin antibody. The scale in FIG. 7 is 50 $\mu$m.

FIG. 8 is a photograph captured when only spheroids are separated and collected, dissociated with a digestive enzyme solution to form single cells, and then cultured in an adherent culture dish containing a neural maintenance medium. Apolar nerve cells, unipolar nerve cells, bipolar nerve cells, and multipolar nerve cells are observed. The scale in FIG. 8 is 100 μm.

FIG. 9 is an analysis result of RT-PCR of cells cultured in an adherent culture dish containing a neural maintenance medium after only spheroids are separated and collected, and dissociated with the digestive enzyme solution to form single cells. Expression of ABCG2, SOX2, and nestin is observed.

FIG. 10 is a result of immunostaining of the small free cells cultured in the adherent culture dish containing a neural maintenance medium after only spheroids are separated and collected, and dissociated with the digestive enzyme solution to form single cells. The presence of nestin positive cells, βIII-tubuhn positive cells, NF 200 positive cells, MAP2 positive cells, MBP positive cells, and GFAP positive cells is observed.

## DETAILED DESCRIPTION OF THE INVENTION

The term "neural cells" herein are cells that constitute a nervous system and include neural stem cells, nerve cells (neurons), glial cells (for example, astrocytes), and the like. The neural cells in the present disclosure preferably include at least neural stem cells.

The term "neural stem cells" herein refer to undifferentiated pluripotent cells in central nervous system, which has pluripotency to differentiate to neurons, glial cells, and the like constituting the brain, spinal cord, and the like, and has self-replication potency. The neural stem cells may be identified with expression of markers such as nestin, SOX2, CD133, and GFAP as indicators.

The term "intraoral mesenchymal tissue" herein is a generic term for connective tissue derived from ectoderm (neural crest) in the oral cavity.

According to one embodiment of the present disclosure, as described above, a method for producing a neural cell-containing preparation includes: preparing an adherent cell culture container in which initially cultured cells derived from intraoral mesenchymal tissue are cultured in a neuron differentiation medium; and collecting cells that do not adhere to the adherent cell culture container from the adherent cell culture container to obtain a cell population, in which the neural cell-containing preparation includes the cell population, or spheroids or neural cells derived from the cell population. Surprisingly, a neural cell-containing preparation that can be used for treatment of nervous tissue can be rapidly produced by the method as described above.

Preparation of initially cultured cells derived from intraoral mesenchymal tissue

According to one embodiment of the present disclosure, the initially cultured cells derived from intraoral mesenchymal tissue can be collected from intraoral mesenchymal tissue. The intraoral mesenchymal tissue preferably includes gingival tissue (such as submucous tissue of the gingiva), submucous tissue of the cheek, buccal fat pad tissue, or dental pulp tissue, and is more preferably dental pulp tissue. The intraoral mesenchymal tissue is preferable from the viewpoint of rapid production of a cell preparation because it can be collected relatively easily from the subject to be treated.

According to a preferred embodiment of the present disclosure, the intraoral mesenchymal tissue is human tissue. The method of the present disclosure is particularly advantageous from the viewpoint of application of the nervous system in the human regenerative medicine field, since the method can be implemented using particularly human tissue as described in examples below.

The method for collecting cells from intraoral mesenchymal tissue is not particularly limited, and can be a method known in the art such as a surgical approach. The intraoral mesenchymal tissue can be advantageously used in human regenerative medicine because it imposes less burden on the patient than, for example, laparotomy or the like.

A method for preparing initially cultured cells of cells collected from intraoral mesenchymal tissue (for example, a culture medium, a culture container, culture conditions, and the like) can be performed by a method known to those skilled in the art. For example, cells obtained by treating and dispersing the intraoral mesenchymal tissue with a protease such as trypsin or EDTA may be suspended in a general basic medium used for cell culture and cultured.

The concentration of cells collected from intraoral mesenchymal tissue in the basic medium is not particularly limited and may be appropriately determined by those skilled in the art. The concentration of cells is, for example, from $1 \times 10^3$ to $1 \times 10^8$ cells/mL, preferably from $1 \times 10^4$ to $1 \times 10^6$ cells/mL, and still more preferably from $1 \times 10^5$ to $1 \times 10^6$ cells/mL.

As the basic medium, Dulbecco's Modified Eagle's Medium (hereinafter referred to as "DMEM"), Iscove's modified Dulbecco's medium (IMDM) (available from Thermo Fisher Scientific, etc.), Ham's F12 medium (F12 medium) (available from SIGMA, Thermo Fisher Scientific, etc.), RPMI 1640 medium, or the like can be used. Two or more basic media may be used in combination. Suitable basic media include DMEM/F12 containing DMEM and F12.

Examples of suitable additives to the basic medium include fetal bovine serum or fetal calf serum (hereinafter referred to as "FBS" or "FCS"), human serum, sheep serum and other sera, serum replacement (such as Knockout serum replacement (KSR)), bovine serum albumin (hereinafter sometimes referred to as "BSA"), antibiotics (such as streptomycin and penicillin), various amino acids (such as non-essential amino acids (NEAAs), and L-glutamine), various vitamins (such as ascorbic acid), various hormones (such as insulin and dexamethasone), and various minerals. As a suitable example, the basic medium may contain DMEM/F12 supplemented with FBS, non-essential amino acids (NEAAs), and L-glutamine. The basic medium and its components described above can also be used in subsequent cell culture steps.

Also, the culture container used for initial culture of the cells collected from intraoral mesenchymal tissue is not particularly limited, and a known culture container can be used. As such a culture container, for example, a container similar to the adherent cell culture container described later may be used.

The cells collected from intraoral mesenchymal tissue can be cultured, for example, for 1 to 10 days, preferably for 1 to 3 days, and more preferably for 1 to 2 days. The culture conditions are not particularly limited, and for example, the cells collected from intraoral mesenchymal tissue may be cultured in a cell culture dish at 37°C, 5% $CO_2$. A culture period may be appropriately determined according to the amount of initially cultured cells required, and the culture may be terminated before reaching confluence in the cell culture dish, or the culture may be terminated after reaching confluence.

As the initially cultured cells, for example, cells subcultured for 1 to 10 passages may be used, and the initially cultured cells are preferably primarily cultured cells or cells subcultured for 2 to 6 passages, more preferably primarily cultured cells or cells subcultured for 2 to 5 passages, still more preferably primarily cultured cells or cells subcultured for 2 to 3 passages, and even more preferably primarily cultured cells. Here, the primarily cultured cells are cells obtained by first seeding cells collected from intraoral mesenchymal tissue and culturing the cells without subculture operation, and can be advantageously used from the viewpoint of obtaining a neural cell-containing preparation at an early stage.

The initially cultured cells derived from intraoral mesenchymal tissue may be treated with, for example, trypsin and EDTA, at 37°C for several minutes, detached from the culture dish, and collected by centrifugation or the like.

Note that the cells collected from intraoral mesenchymal tissue may be cultured as they are in the neuron differentiation medium described later after collection without being cultured in a general basic medium as described above. Therefore, the "initially cultured cells derived from intraoral mesenchymal tissue" in the present disclosure include both cells cultured in the neuron differentiation medium after being cultured with a basic medium and cells cultured in the neuron differentiation medium without being cultured with a basic medium. According to one embodiment, the initially cultured cells derived from intraoral mesenchymal tissue are primarily cultured cells that are cultured in the neuron differentiation medium without being cultured with a basic medium.

Culture of initially cultured cells derived from intraoral mesenchymal tissue in neuron differentiation medium

According to one embodiment, in the method of the present disclosure, the initially cultured cells derived from intraoral mesenchymal tissue are cultured in an adherent cell culture container containing a neuron differentiation medium.

As the neuron differentiation medium in this step, a known neuron differentiation medium can be used, and the neuron differentiation medium may contain the basic medium and an additive thereof. Preferably, the neuron differentiation medium contains a basic medium containing DMEM/F12.

The neuron differentiation medium of the present disclosure preferably contains a neural differentiation inducing factor. The neural differentiation inducting factor in the present disclosure is not particularly limited, and is preferably a factor that induces signaling associated with neural differentiation. According to one embodiment, the neural differentiation inducting factor may be a retinoid that stimulates retinoid signaling in human pluripotent stem cells. Suitable retinoids include retinol, all-trans retinoic acid (ATRA), retinol acetate, and the like, and all-trans retinoic acid is preferable. The content of the retinoid in the neuron differentiation medium is, for example, from 0.01 nM to 10 $\mu$M, preferably from 0.01 nM to 100 nM, more preferably from 0.1 nM to 20 nM, still more preferably from 1 nM to 15 nM, and even more preferably from 5 nM to 15 nM.

In addition, it is preferable that the primary placenta-associated secretory product in the present disclosure are added to the neuron differentiation medium from the viewpoint of stably culturing the initially cultured cells under an environment similar to that of the primary placental cells. Thus, according to one embodiment, the neuron differentiation medium contains all-trans retinoic acid and primary placenta-associated secretory products. Suitable primary placental cell-associated secretory products include progesterone, estradiol, NGF-1, triiodothyronine (T3), thyroxine (T4), or the like. According to a more preferable embodiment, the neuron differentiation medium contains all-trans retinoic acid and NGF-1. The total content of the primary placenta-associated secretory products in the neuron differentiation medium is, for example, from 0.01 nM to 10 $\mu$M, preferably from 0.01 nM to 100 nM, more preferably from 0.1 nM to 80 nM, still more preferably from 30 nM to 80 nM, and even more preferably from 45 nM to 65 nM.

The content of progesterone in the neuron differentiation medium is, for example, from 0.01 nM to 100 nM, more preferably from 0.1 nM to 30 nM, and still more preferably from 10 nM to 25 nM. The content of estradiol in the neuron differentiation medium is, for example, from 0.01 nM to 100 nM, more preferably from 0.1 nM to 30 nM, and still more preferably from 10 nM to 25 nM. The content of NGF-1 in the neuron differentiation medium is, for example, from 0.01 nM to 100 nM, more preferably from 0.1 nM to 30 nM, and still more preferably from 5 nM to 15 nM. The content of thyroxine in the neuron differentiation medium is, for example, from 0.01 ng/ml to 100 ng/ml, more preferably from 0.1 ng/ml to 30 ng/ml, and still more preferably from 5 nM to 15 nM.

According to one embodiment, the neuron differentiation medium may contain at least one or all of NEAAs, antibiotics (such as streptomycin, penicillin, and fungizone), various amino acids (such as non-essential amino acids (NEAAs) and L-glutamine), various vitamins (such as ascorbic acid), and various hormones (such as insulin and dexamethasone), in addition to the neural differentiation inducting factor and the primary placental cell-associated secretory products. A preferable example of the neuron differentiation medium of the present disclosure is a medium described in Takahashi H, Ishikawa H, Tanaka A. Regenerative medicine for Parkinson's disease using differentiated nerve cells derived from human buccal fat pad stem cells. Human Cell DOI 10.1007/s13577-017-0160-3, 2017. The content of each component in the neuron differentiation medium can be appropriately set by those skilled in the art according to the state of the initially cultured cells and the like.

The concentration of the initially cultured cells added to the neuron differentiation medium is not particularly limited and may be appropriately determined by one skilled in the art, and is, for example, from $1 \times 10^3$ to $1 \times 10^8$ cells/60 mm dish, preferably from $1 \times 10^4$ to $1 \times 10^6$ cells/60 mm dish, and still more preferably from $1 \times 10^5$ to $1 \times 10^6$ cells/60 mm dish.

The culture temperature of the initially cultured cells in the neuron differentiation medium is, for example, from 30°C to 40°C, preferably about 37°C, and the $CO_2$ concentration is, for example, from 1% to 10%, and preferably about 5%.

In addition, according to one embodiment of the present disclosure, the container surface in contact with the initially cultured cell in the adherent cell culture container is preferably adhesive to the neural cells of which the axons are elongated. Here, the term "adherent" refers to a characteristic of the container surface as a scaffold to which the cultured cells can adhere, and for example, the container surface can be made adherent by surface oxidation/hydrophilization such as a plasma treatment, a corona discharge treatment, an oxidizing agent treatment, or a hydrophilic substance coating treatment with polylysine or the like. In addition, for example, when an adherent basement membrane matrix such as Matrigel (trade name, available from BD Falcon) is used, a cell-adherent coating can be applied to its adhesion surface.

The initially cultured cells in the neuron differentiation medium can be cultured for 0.5 to 10 days, preferably for 0.5 to 3 days, and more preferably for 1 to 2 days.

According to one embodiment of the present disclosure, in accordance with the description above, an adherent cell culture container in which the initially cultured cells derived from intraoral mesenchymal tissue are cultured in the neuron differentiation medium is prepared according to the above description. The preparation of the adherent cell culture container of the present disclosure includes not only an aspect of producing the adherent cell culture container in which the initially cultured cells derived from intraoral mesenchymal tissue are cultured in the neuron differentiation medium, but also an aspect of purchasing the above adherent cell culture container.

In one embodiment of the present disclosure, cells that do not adhere to the adherent cell culture container are collected from the neuron differentiation medium. The collected cells are preferably free cells present without adhering to the surface of the adherent cell culture container or cells further adhering to the cells that adhere to the adherent cell culture container, more preferably free cells present in the neuron differentiation medium or cells further adhering to the cells that adhere to the adherent cell culture container, and still more preferably free cells present in the neuron differentiation medium.

According to one embodiment of the disclosure, the collected cells have an average diameter from 10 to 20 $\mu$m, and more preferably from 12 to 16 $\mu$m. Here, the average diameter of the collected cells can be measured by an optical microscope. Specifically, a photograph is captured by an optical microscope from directly above the adherent cell culture container, the photograph is divided into four parts of the same area, arbitrary 25 cells (100 cells in total in the photograph) are selected for each divided part, the average of the longest diameter and the shortest diameter is calculated as the cell diameter of one cell, and the average of cell diameters of 100 cells is calculated as the average diameter.

Further, the ratio of the longest diameter to the shortest diameter (longest diameter/shortest diameter) of the collected cells is not particularly limited, and is preferably from 1 to 1.5, and more preferably from 1 to 1.2. The ratio of the longest diameter to the shortest diameter can be measured in accordance with the method for measuring the average diameter described above.

Also, the shape of the cells collected is not particularly limited, and is, for example, suitably spherical or elliptical. The shape of the cells collected can be identified by optical microscopy.

Collection of the cells to obtain a cell population may be performed by any specific means as long as the cells are separated and collected in such a manner that the functions thereof are not reduced. Such collection can be performed by methods known to those skilled in the art. According to a preferable embodiment in the present disclosure, the method can be performed by centrifuging the cells suspended in the neuron differentiation medium. According to the other preferable embodiment in the present disclosure, the method can be performed by aspirating the cells suspended in the neuron differentiation medium with a micropipette under a microscope. Furthermore, according to the other preferable embodiment of the present disclosure, the method can be performed by a magnetic bead method, a FACS method, or the like using an antibody that recognizes a surface antigen specifically expressed/not specifically expressed on the surface of the cell.

According to one embodiment of the present disclosure, the cell population obtained by collection contains high levels of neural cells, as is apparent from examples described later. According to one embodiment, the cell population preferably contains neural stem cells.

The neural cells included in the cell population can be confirmed by the presence or absence of expression of a cell marker. Examples of such markers include nestin, βIII-tubulin, Nanog, Oct 3/4, ABCG2, SOX2, NF200, MAP2, MBP, and GFAP. Thus, according to one embodiment, the cell population contains cells expressing at least one cell marker selected from the group consisting of nestin, βIII-tubulin, Nanog, Oct 3/4, ABCG2, SOX2, NF200, MAP2, MBP, and GFAP. According to a more preferable embodiment, the cell population contains cells expressing at least one cell marker selected from the group consisting of nestin, βIII-tubulin, Nanog, Oct 3/4, and SOX2. According to a still more preferable embodiment, the cell population contains cells expressing nestin, βIII-tubulin, Nanog, Oct 3/4, and SOX2.

In the cell population of the present disclosure, the proportion of high levels of the neural cells included in the cell population is not particularly limited, and is, for example, from 50% to 100%, preferably from 70% to 100%, more preferably from 75% to 100%, and still more preferably from 80% to 100%. The proportion of the neural cells is calculated from the number of cells involved in the formation of spheroids in spheroid formation according to the description of examples below. Specifically, the proportion of the neural cells in the cell population can be calculated by the following equation.

[Equation 1]

[Proportion of neural cells included in cell population] = [(total number of cells in cell population)-(number of cells not forming spheroid)]/(total number of cells in cell population) × 100

The cell population of the present disclosure can be particularly advantageously utilized for subjects in need of early treatment because it is acquired in a short time despite containing high levels of neural cells.

Spheroid formation

According to one embodiment, the method of the present disclosure further includes spheroid formation in which the cell population is cultured to form spheroids. Since the cell population of the present disclosure contains high levels of the neural cells, the spheroids can be formed by self-assembly of the neural cells, and the spheroids can be advantageously used for easily separating and purifying the neural cells from other cells.

The method for culturing a cell population in the spheroid formation is not particularly limited, and from the viewpoint of promoting contact between cells to efficiently produce a spheroid, it is preferable to use a three-dimensional culture method performed under conditions of non-adhesion to a culture container. Specifically, the method for culturing the cell population is preferably suspension culture, stirring culture, rotary culture, or shaking culture, and more preferably rotary culture.

The cell-contacting surface of the culture container used for culturing the cell population in the spheroid formation is preferably non-adherent from the viewpoint of promoting cell contact due to physical disturbance as described above to efficiently produce spheroids. Here, the term non-adherent refers to a characteristic of a container surface to which cultured cells cannot adhere as a scaffold, and for example, the container surface can be made non-adherent by hydrophobic treatment, coating treatment of a non-adherent material, or the like. The non-adherent coating is not particularly limited as long as it is a coating having cell non-adhesiveness, and examples thereof include coatings of celluloses such as methyl cellulose, hydroxyethyl cellulose, hydroxypropyl cellulose, hydroxypropyl methyl cellulose, or sodium carboxymethyl cellulose; polyethylene oxide; carboxyvinyl polymer; polyvinylpyrrolidone; polyethylene glycol; polylactic acid; polyamides such as polyacrylamide and poly N-isopropylacrylamide; polysaccharides such as chitin, chitosan, hyaluronic acid, alginic acid, starch, pectin, carrageenan, guar gum, gum arabic, and dextran, and cell non-adhesive derivatives thereof.

The culture medium of the cell population in the spheroid formation is not particularly limited as long as spheroid formation is not hindered, and a commercially available medium used as a neural maintenance medium can be used. Specifically, examples of the medium that can be used in this step include the basic medium described above including DMEM/F12, N-GRO (DV Biologics), and B-27 Plus Neuronal Culture System (Thermo Fisher Scientific). According to one embodiment, the culture medium of the cell population in the spheroid formation is a serum-containing medium. The serum concentration in the serum medium is preferably about from 1% to 20%, and more preferably about from 5% to 10%. The serum concentration may be increased stepwise within this range or may be constant. In addition, a neurotrophic factor that promotes axon elongation can be added to the neural maintenance medium. Examples of the neurotrophic factor include BDNF and Neurotrophins-3 and -4, and BDNF is preferable. In addition, other growth factors (for example, FGF or the like) and additives (N2 supplement) may be contained. However, when the addition of retinoid (retinoic acid or the like) to the neural maintenance medium is continued, axon elongation is inhibited after a lapse of a time period even if the axon is elongated, and thus it is preferable that the neural maintenance medium does not contain retinoid.

The culture temperature in the spheroid formation is, for example, from 30°C to 40°C, preferably about 37°C, and the $CO_2$ concentration is, for example, from 1% to 10%, and preferably about 5%.

The period for culturing the cell population in the spheroid formation is not particularly limited, and is, for example, from 0.5 to 2 days, preferably from 0.5 to 1.5 days, and more preferably 1 day from the viewpoint of rapid application of the spheroid to a cell preparation.

Differentiation promotion

According to one embodiment, the method of the present disclosure further includes dispersing and culturing the spheroids. Performing such a step is preferable from the viewpoint of promoting differentiation of cells constituting spheroids into the nervous system.

The method for dispersing the spheroids is not particularly limited, and can be performed using a known method. For example, the spheroids may be dispersed by treatment with a protease such as trypsin and EDTA.

The culture medium in the spheroid dispersion culture is not particularly limited as long as the formation of the neural cells is not hindered, and for example, a neural maintenance medium similar to that in the spheroid formation can be used. From the viewpoint of promoting differentiation into neural cells, the above neuron differentiation medium may be used in this step, and the present disclosure also includes such an aspect.

The concentration of the spheroid-derived cells in the medium in the spheroid dispersion culture is not particularly limited, and may be appropriately determined by those skilled in the art, and is, for example, from $1 \times 10^3$ to $1 \times 10^8$ cells/mL, preferably from $1 \times 10^4$ to $1 \times 10^6$ cells/mL, and still more preferably from $1 \times 10^5$ to $1 \times 10^6$ cells/mL.

The culture temperature in the spheroid dispersion culture is, for example, from 30°C to 40°C, preferably about 37°C, and the $CO_2$ concentration is, for example, from 1% to 10%, and preferably about 5%.

The culture period of the spheroid-derived cells in the spheroid dispersion culture is not particularly limited, and is, for example, from 1 to 7 days, preferably from 1 to 5 days, and more preferably from 1 to 4 days from the viewpoint of rapid application of the spheroid to a cell preparation. In the step of obtaining the above neural cells, it is preferable to perform subculture from the viewpoint of securing the amount of the neural cells applicable to the treatment.

The presence of the neural cells in the spheroids can be confirmed by the presence or absence of expression of a marker of cells obtained by dispersion culture. Examples of such markers include nestin, βIII-tubulin, Nanog, Oct 3/4, ABCG2, SOX2, NF200, MAP2, MBP, and GFAP. Thus, according to one embodiment, the neural cells or cell population thereof of the present disclosure express at least one cell marker selected from the group consisting of nestin, βIII-tubulin, Nanog, Oct 3/4, ABCG2, SOX2, NF200, MAP2, MBP, and GFAP. According to a more preferable embodiment, the neural cells or cell population thereof of the present disclosure express at least one cell marker selected from the group consisting of nestin, βIII-tubulin, ABCG2, SOX2, NF200, MAP2, MBP, and GFAP. According to a still more preferable embodiment, the neural cells or cell population thereof of the present disclosure express at least one cell marker selected from the group consisting of nestin, βIII-tubulin, NF200, MAP2, MBP, and GFAP.

In addition, according to the method of the present disclosure, it is possible to very easily perform a process from the start of the primary culture to the acquisition of the neural cell-containing preparation. According to a preferable embodiment of the present disclosure, the period from the start of the primary culture to the acquisition of the cell preparation is preferably from 2 to 10 days, more preferably from 3 to 8 days, and still more preferably from 5 to 7 days.

Neural cell population

The method of the present disclosure can be advantageously utilized in suppressing the occurrence of chromosomal abnormalities and providing a neural cell population. Without being bound by theory, it is believed that the occurrence rate of chromosomal abnormalities can be reduced at high levels because the culture period can be shortened and exposure of cells to chemicals can be suppressed.

According to one embodiment of the present disclosure, there is provided a neural cell population derived from intraoral mesenchymal tissue cells, wherein a proportion of normal diploid cells is 80% or more. Here, the term "normal diploid" refers to a cell having normal diploid nature having, for example, 46 chromosomes in human cells. The proportion of the normal diploid can be determined as an average according to the method described in Test Example 2, which will be described later. The proportion of the normal diploid cells in the neural cell population of the present disclosure is preferably 90% or more, more preferably 95% or more, still more preferably 98% or more, even more preferably 99% or more, and even still more preferably 100%.

In addition, the derivative tissues, the number of passages, the expression of markers, and the production method regarding the neural cell population of the present disclosure are as described above.

Neural cell-containing preparation

According to one embodiment of the present disclosure, a neural cell-containing preparation can be efficiently obtained using the above method. Since the neural cell-containing preparation of the present disclosure can be produced in a very short time, it can be advantageously utilized in the rapid treatment of nervous tissue.

The neural cell-containing preparation can contain neural cells in a desired differentiation state and form by appropriate selection of culture conditions and cell collection time in the above production method. Therefore, the neural cells in the neural cell-containing preparation may be in any form of single cells, a cell population or spheroids, an organ tissue or a part thereof, or a mixture of the cell and another cell or a substance other than cells. In addition, the neural cell-containing preparation may contain any of neural stem cells, nerve cells, and glial cells, and preferably contains neural stem cells.

The cell numbers of the cell population contained in the neural cell-containing preparation, or the cell numbers of spheroids or the neural cells derived from the cell population can be appropriately adjusted according to the nature or condition of the administration target, and can be, for example, from 50 to $1 \times 10^{10}$ cells/mL, and preferably from $1 \times 10^4$ to $1 \times 10^8$ cells/mL.

The content of the neural cells in the neural cell-containing preparation is not particularly limited, and may be appropriately determined according to a state of the patient and a state of the cells. The content of the neural cells in the neural cell-containing preparation can be, for example, from 0.1 to 100 mass%, and is preferably from 50 to 100 mass%.

In addition, the neural cell-containing preparation of the present disclosure may contain serum albumin or the like from the viewpoint of protecting cells. In addition, the neural cell-containing preparation may contain an antibiotic or the like from the viewpoint of preventing contamination and proliferation of bacteria. In addition, the neural cell-containing preparation may contain other components (for example, a carrier, an excipient, a disintegrant, a buffer, an emulsifier, a suspension, a soothing agent, a stabilizer, a preservative, an antiseptic, physiological saline, and the like) acceptable in the preparation, or cells other than the neural stem cells obtained in the present disclosure.

Since the neural cell-containing preparation of the present disclosure can be produced in a very short time as described above, it can be advantageously used in medical care for the purpose of early repair of nervous system tissue. Thus, according to one embodiment of the disclosure, the neural cell preparation is employed for repair of nervous tissue. Suitable examples of nervous tissue include the following. First, the brain includes all the central nerve of the brains in addition to cerebral grey matter, cerebral white matter, basal ganglia, limbic system, thalamus, midbrain, pons, medulla, and cerebellum. Next, examples of the neural cells include neural cells, neuroglia cells, microglia cells, astrocytes, neural cells that release neurotransmitters including catecholamine such as dopamine and hormones, and anterior pituitary gland and posterior pituitary gland. The spinal cord includes all the central nerves of spinal cords such as cervical spinal cord, thoracic spinal cord, lumbar spinal cord, sacral spinal cord, and cauda equina. The peripheral nerve is all nerves existing in each part of the body other than the central nerve, and specifically includes the first to XIIth cranial nerves (olfactory nerve, optic nerve, oculomotor nerve, trochlear nerve, trigeminal nerve, abducens nerve, facial nerve, auditory nerve, glossopharyngeal nerve, vagus nerve, accessory nerve, and hypoglossal nerve) which are nerves originating from the brain. There are the first cervical spinal cord to the eighth cervical spinal cord, the first thoracic spinal cord to the twelfth thoracic spinal cord, the first lumbar spinal cord to the fifth lumbar spinal cord, and the first sacral spinal cord to the fifth sacral spinal cord, which are nerves originating from the spinal cord, and radial nerve, median nerve, ulnar nerve, axillary nerve, femoral nerve, obturator nerve, peroneal nerve, tibial nerve, and sciatic nerve which are the peripheral nerves of the spinal cord. Other peripheral nerves include mandibular nerve, recurrent nerve, phrenic nerve, and the like. These nerves include all nervous systems such as motor nerve, sensory nerve, sympathetic nerve, parasympathetic nerve, and the like.

In addition, the neural cell-containing preparation of the present disclosure can be used as a medicine or a material for regenerative medicine for repairing a nervous tissue in various forms in accordance with the therapy. According to a preferable embodiment, the neural cell-containing preparation is provided as a pharmaceutical preparation. According to a more preferable embodiment, the neural cell-containing preparation is provided as a preparation for regenerative medicine. According to the other embodiment, the neural cell-containing preparation is provided as a transplantation material or a tissue for transplantation. In addition, according to one embodiment, the neural cell-containing preparation is provided as a mixture of cells produced according to the present disclosure and other cellular material or non-cellular material. According to a more preferable embodiment, the neural cell-containing preparation is used for autologous transplantation. In addition, according to one embodiment, the neural cell-containing preparation is used for the treatment of an acute neurological disease or the repair of nervous tissue damage.

In addition, the neural cell-containing preparation of the present disclosure is preferably a preparation for autologous transplantation or allogenic transplantation, more preferably a preparation for allograft autologous transplantation or allograft allogenic transplantation, and still more preferably for allograft autologous transplantation. The neural cell-containing preparation can be used for either autologous transplantation or allogenic transplantation, but is preferably used for autologous transplantation from the viewpoint of avoiding transplantation rejection and infection.

The cell population of the present disclosure or spheroids or neural cells derived from the cell population can be advantageously utilized in the repair of nervous tissue involved in nervous system disease, as described above. Therefore, according to the other embodiment, there is provided a method of treating a nervous system disease, the method including administering an effective amount of the cell population of the present disclosure, or spheroids or neural cells derived from the cell population to a subject in need thereof. Here, the term "treatment" includes not only treating established pathologies but also preventing pathologies that may be established in the future.

The subject is a mammal, for example, a rodent, a dog, a cat, a cow, a primate, or the like, and is preferably a human. In addition, examples of the nervous system disease include all the brain, spinal cord, and peripheral nerve damages, and specific examples of the brain disease include stroke and cerebrovascular disorders such as cerebral infarction, cerebral hemorrhage, Moyamoya disease, and subarachnoid hemorrhage, traumatic brain damages such as cerebral contusion and diffuse axonal injury, hypoxic encephalopathy, encephalopathy associated with carbon monoxide intoxication and drug intoxication, encephalitis, brain abscess, encephalopathy associated with infection, cerebral palsy, periventricular leukomalacia, progressive multifocal leukoencephalopathy, sub-acute sclerosing panencephalitis, Alzheimer's disease, dementia with Lewy bodies, epilepsy associated with cortical dysplasia, and Parkinson's disease. Examples of the spinal cord and peripheral nerve diseases include spinal cord injury, spinal cord infarction, spinal cord edema, syringomyelia, degenerative myelopathy, spondylotic myelopathy, brachial plexus injury, mandibular nerve palsy, recurrent laryngeal nerve palsy, phrenic nerve palsy, brachial plexus injury, sciatic nerve palsy, femoral nerve palsy, spinocerebellar degeneration, amyotrophic lateral sclerosis, spinal muscular atrophy, spinal and bulbar muscular atrophy, Guillain-Barr syndrome, chronic inflammatory demyelinating polyneuropathy, hereditary neuropathy, and Charcot-Marie-Tousse disease, and spinal cord injury and stroke are preferable.

According to one embodiment, the cell population of the present disclosure or spheroids or the neural cells derived from the cell population can be administered to a subject by known administration routes and techniques. For example, in addition to intravenous injection, intramuscular injection, subcutaneous administration, rectal administration, transdermal administration, topical ocular administration, and transpulmonary administration, it can be directly administered to an affected area (spinal cord, brain, various nervous systems) in which damage of the neural cells has occurred.

The effective amount of the cell population or the spheroids or the neural cells derived from the cell population is not particularly limited, and can be appropriately set according to the type of the nervous system disease, the nature or condition of the subject, the number of administrations, and the like, and for example, can be from $1 \times 10^4$ to $1 \times 10^8$ cells per administration. In addition, the number of administrations and the administration interval of the cell population or the spheroids or the neural cells derived from the cell population are not particularly limited, and can be appropriately set by those skilled in the art according to the nature or condition of the subject. The number of administrations may be, for example, from 1 to 10 times, and preferably from 1 to 3 times per day. The administration interval may be, for example, every 1 day to 6 months, and preferably every 1 day to 1 month.

Further, according to the other embodiment, there is provided a cell population of the present disclosure, or spheroids, neural cells or a nervous system tissue derived from the cell population, or use thereof as a neural cell-containing preparation. Further, according to the other embodiment, there is provided a cell population of the present disclosure, or spheroids, neural cells or a nervous system tissue derived from the cell population, or use thereof as a neural cell-containing preparation for repairing nervous tissue. Further, according to the other preferable embodiment, there is provided a cell population of the present disclosure, or spheroids, neural cells or a nervous system tissue derived from the cell population, or use thereof as a neural cell-containing preparation for regenerative medicine. Further, according to the other preferable embodiment, there is provided a cell population of the present disclosure, or spheroids, neural cells or a nervous system tissue derived from the cell population, or use thereof as a neural cell-containing preparation used as a transplantation material. Further, according to the other preferable embodiment, there is provided a cell population of the present disclosure, or spheroids, neural cells or a nervous system tissue derived from the cell population, or use thereof as a neural cell-containing preparation used as an autologous transplantation material.

Further, according to the other embodiment, there is provided use of a cell population of the present disclosure, or spheroids, neural cells or a nervous system tissue derived from the cell population in the production of a neural cell-containing preparation. Further, according to the other embodiment, there is provided use of a cell population of the present disclosure, or spheroids, neural cells or a nervous system tissue derived from the cell population in the production of a preparation for repairing nervous tissue. Further, according to the other preferable embodiment, there is provided use of a cell population of the present disclosure, or spheroids, neural cells, or a nervous system tissue derived from the cell population in the production of a neural cell-containing preparation for regenerative medicine. Further, according to the other preferable embodiment, there is provided use of a cell population of the present disclosure, or spheroids, neural cells or a nervous system tissue derived from the cell population in the production of a neural cell-containing preparation used as a transplantation material. Further, according to the other preferable embodiment, there is provided use of a cell population of the present disclosure, or spheroids or neural cells or a nervous system tissue derived from the cell population in the production of a neural cell-containing preparation used as an autologous transplantation material.

According to one embodiment, the present disclosure includes the following (1) to (31).

(1) A method for producing a neural cell-containing preparation, the method including:

preparing an adherent cell culture container in which initially cultured cells derived from intraoral mesenchymal tissue are cultured in a neuron differentiation medium; and
collecting cells that do not adhere to the adherent cell culture container from the adherent cell culture container to obtain a cell population,
wherein the neural cell-containing preparation includes the cell population, or spheroids or neural cells derived from the cell population.

(2) The method according to (1), wherein a period from start of the initial culture to acquisition of the neural cell-containing preparation is from 2 to 10 days.
(3) The method according to (1) or (2), wherein the cells that do not adhere to the adherent cell culture container are free cells present in the neuron differentiation medium or cells further adhering to cells that adhere to the adherent cell culture container.
(4) The method according to any one of (1) to (3), wherein the intraoral mesenchymal tissue is selected from the group consisting of gingival tissue, submucous tissue of cheek, buccal fat pad tissue, and dental pulp tissue.
(5) The method according to any one of (1) to (4), wherein the intraoral mesenchymal tissue is human tissue.
(6) The method according to any one of (1) to (5), wherein the initially cultured cell period is primarily cultured cells or cells subcultured for 2 to 5 passages.
(7) The method according to any one of (1) to (6), wherein the cells that do not adhere to the adherent cell culture container have an average diameter from 12 to 16 $\mu$m.
(8) The method according to any one of (1) to (7), wherein a period during which the initially cultured cells are cultured in the neuron differentiation medium is from 0.5 to 10 days.
(9) The method according to any one of (1) to (8), wherein a cell-contacting surface of the container containing the neuron differentiation medium is adhesive to neural cells of which axons are elongated.
(10) The method according to any one of (1) to (9), wherein collection of the free cells is performed by centrifugation, aspiration, a magnetic bead method, or a FACS method.
(11) The method according to any one of (1) to (10), further including: culturing the cell population to form spheroids.
(12) The method according to (11), wherein a period for culturing the cell population in the spheroid formation is from 0.5 to 2 days.
(13) The method according to (10) or (12), wherein a cell-contacting surface of a container used for culturing the cell population in the spheroid formation is non-adherent to neural cells of which axons are elongated.
(14) The method according to any one of (10) to (12), wherein the culture of the cell population in the spheroid formation is performed by suspension culture, stirring culture, rotary culture, or shaking culture.
(15) The method according to any one of (10) to (14), further including: dispersing and culturing cells from the spheroids.
(16) The method according to (15), wherein a culture period of cells obtained by dispersing the spheroid is from 3 hours to 7 days.
(17) The method according to any one of (1) to (16), wherein the cell preparation contains neural stem cells.
(18) The method according to any one of (1) to (17), wherein the neural cell-containing preparation is a preparation for regenerative medicine.
(19) The method according to any one of (1) to (18), wherein the neural cell-containing preparation is a preparation for autologous transplantation or allogenic transplantation.
(20) The method according to any one of (1) to (19), wherein the cell preparation is used for repairing nervous tissue damage.
(21) A neural cell-containing preparation obtained by the method described in any one of (1) to (20).
(22) A neural cell population derived from intraoral mesenchymal tissue cells, wherein a proportion of normal diploid cells is 80% or more.
(23) The neural cell population according to (22), wherein the proportion of the normal diploid cells is 90% or more.

(24) The neural cell population according to (22) or (23), wherein the intraoral mesenchymal tissue cells are primarily cultured cells or cells subcultured for 2 to 5 passages.

(25) The neural cell population according to any one of (22) to (24), wherein the intraoral mesenchymal tissue is selected from the group consisting of gingival tissue, submucous tissue of cheek, buccal fat pad tissue, and dental pulp tissue.

(26) The neural cell population according to any one of (22) to (25), wherein the intraoral mesenchymal tissue is human tissue.

(27) The neural cell population according to any one of (22) to (26), which is obtained by the method described in (15).

(28) A pharmaceutical preparation containing the neural cell population described in any one of (22) to (27).

(29) The pharmaceutical preparation according to (28), which is a preparation for regenerative medicine.

(30) The pharmaceutical preparation according to (28) or (29), which is a preparation for autologous transplantation or allogenic transplantation.

(31) The pharmaceutical preparation according to (30), which is used for repairing nervous tissue damage.

## EXAMPLES

The present disclosure will be described in detail using examples, but the present disclosure is not limited to these examples.

## Materials and Analytical methods

## Collection of ectodermal mesenchymal tissue

After obtaining an informed consent of the patient, ectodermal mesenchymal tissue (submucous tissue of the gingiva, submucous tissue of the cheek, buccal fat pad tissue, or dental pulp tissue) was collected from the patient. These tissues were washed with a sufficient amount of Hanks' solution and then minced with a razor blade.

## Marker analysis

The following techniques were used to characterize the cells obtained in the test examples described below.

## Detection of cell characteristic markers by immunostaining

The cells to be analyzed were seeded at $1 \times 10^5$ cells/well on 4-well chamber slides (available form Iwaki or Nunc) pre-coated with Matrigel (trade name) basement membrane. For immunostaining, the cells were immobilized at -30°C for 15 minutes using methanol (available from FUJIFILM Wako Pure Chemical Corporation), and then incubated at room temperature for 10 minutes using Blocking One Histo (available from Nacalai Tesque, Inc.). Cells were then incubated overnight at 4°C with the primary antibodies described in Table 1 below. Cells treated with primary antibodies were then treated with a secondary antibody of Alexa Fluor 488 conjugated goat anti-rabbit IgG, Alexa Fluor 488 conjugated goat anti-mouse IgG, and Alexa Fluor 488 conjugated goat anti-chicken IgG (available from Thermo Fischer Scientific, all of which were used at a dilution rate of 1 : 1000), and incubated in the dark at room temperature for 30 minutes. Nuclei were stained with VECTASHIELD HardSet (trade name) mounting medium (available from Vector Laboratories) using DAPI. Images of the cells were acquired using a confocal laser microscope (available from Carl Zeiss, model number: LSM-510).

Some of the antibodies used for immunostaining are as described in Table 1 below.

[Table 1]

Table 1. Antibodies used for immunostaining

| Antibody | Source (Catalog No.) | Dilution rate |
|---|---|---|
| Anti-βIII-tubulin (T U J 1) | Covance (MMS-435P) | 1:500 |
| Anti-nestin | Merck (N5413) | 1:500 |
| Anti-MAP2 | Merck (M4403) | 1:500 |
| Anti-NF200 | Merck (MAB5262) | 1:1000 |

(continued)

| Antibody | Source (Catalog No.) | Dilution rate |
|----------|---------------------|---------------|
| Anti-GF AP | Dako (NI506, NP043) | 1:500 |
| Anti-MBP | Abcam (ab62631) | 1:1000 |

Detection of intracellularly expressed gene marker by RT-PCR method

Total RNA was extracted from cells using Trizol reagent (available from Thermo Fischer Scientific). cDNA was synthesized from 1 $\mu$g of total RNA using High-Capacity cDNA reverse transcription kit (available from Thermo Fischer Scientific). Gene amplification was performed using PCR Supermix Platinum kit (available from Thermo Fischer Scientific) and Veriti (trade name) 96-well thermal cycler. Primers and annealing temperatures for each gene are shown in Table 2 below. PCR products were analyzed by 2% (w/v) agarose gel electrophoresis and ethidium bromide staining.

[Table 2]

Table 2. Primers and annealing temperatures for each gene

| Gene name | SEQ ID NO. (5'-3') | Tm | Size (base pairs) | Accession No. |
|-----------|--------------------|----|-----|---------------|
| ABCG2 | F: ACCATTGCATCTTGGCTGTC (SEQ ID NO: 1) R:CGATGCCCTGCTTTACCAAA (SEQ ID NO: 2) | 56.5 | 181 | NM_001257386 |
| Sox2 | F: AACCCCAAGATGCACAACTC (SEQ ID NO: 3) R: CGGGGCCGGTATTTATAATC (SEQ ID NO: 4) | 60 | 152 | NM_003106 |
| Nestin | F: AACAGCGACGGAGGTCTCTA (SEQ ID NO: 5) R:TTCTCTTGTCCCGCAGACTT(SEQ ID NO: 6) | 55 | 220 | NM_006617 |

Test Example 1: Method for preparing differentiation-induced neural cells from dental pulp tissue

A neural cell population differentiated and induced from neural stem cells derived from human dental pulp tissue was obtained by the method described in the following (1) to (4).

(1) Initial culture

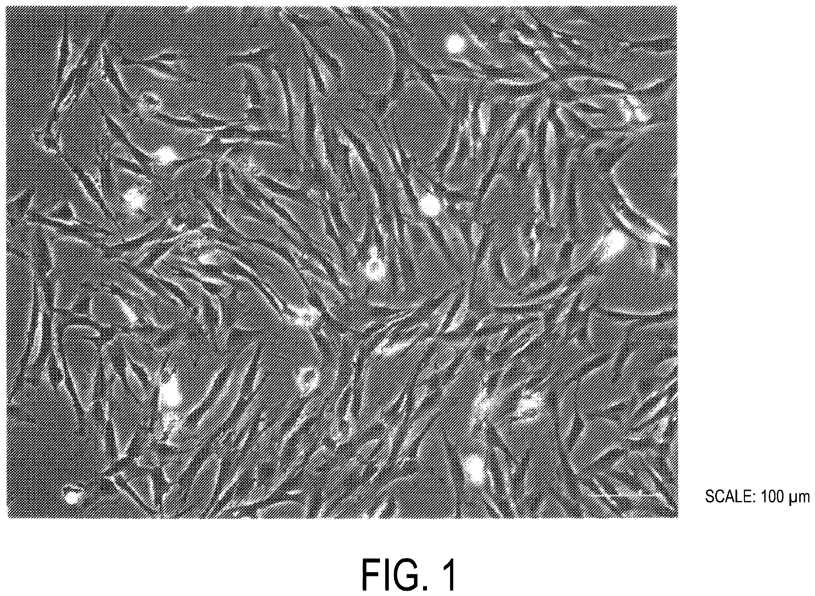

The sliced dental pulp tissue was incubated in a 0.25% trypsin, 0.02% EDTA/PBS (-) solution at 37°C for 30 minutes, then a small amount of fetal bovine serum (FBS) was added, and then the cells were dispersed by pipetting. The dispersed cells were centrifuged at 340 $\times$ g for 5 minutes, and the precipitate was resuspended in a growth medium (DMEM/F12 supplemented with 10% fetal bovine serum (FBS), 100 $\mu$M glutaMAX, 0.1% non-essential amino acids (NEAAs)). The resuspended liquid was seeded on a 5 $\times$ 10$^5$ cells/60 mm culture dish (3 mL culture solution), and cultured at 37°C and 5% carbon dioxide for 3 to 48 hours to obtain initially cultured cells (primarily cultured cells).
As a result, the initially cultured cells derived from human dental pulp were elongated cells (FIG. 1).

(2) Free cell generation and separation and collection thereof

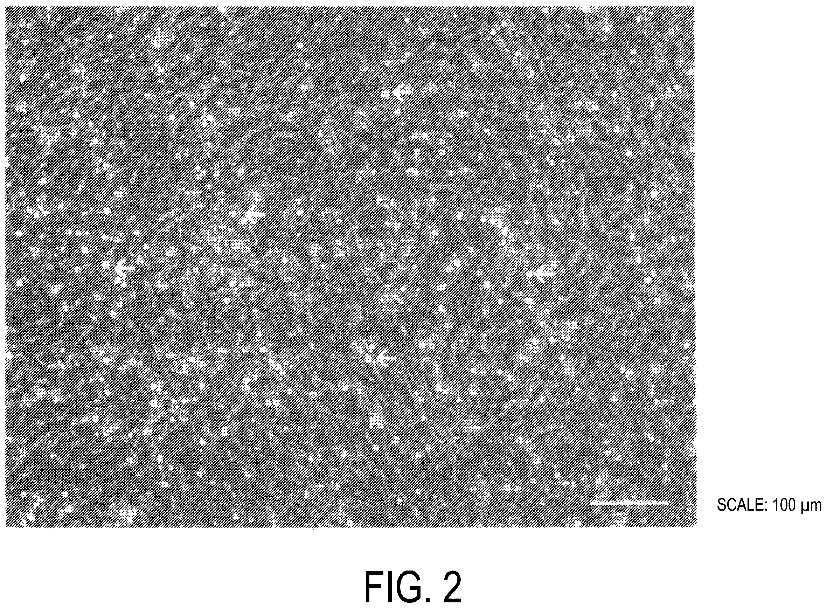

Initially cultured cells (5 $\times$ 10$^5$ cells/60 mm culture dish) of human dental pulp tissue were seeded in an adherent culture dish (60 mm, product name: Falcon 353802), and then cultured in a neuron differentiation medium (DMEM/F12 supplemented with 5% fetal bovine serum (FBS), 10 $\mu$M non-essential amino acids (NEAAs), 2 mM glutaMAX, 10 nM all-trans retinoic acid (ATRA), 50 $\mu$M ascorbic acid, 5 $\mu$M insulin, 10 nM dexamethasone, 20 nM progesterone, 20 nM estradiol, 10 nM NGF-1, 10 ng/ml thyroxine (T4), 50 U/ml penicillin, 50 pg/ml streptomycin, and 0.25 pg/ml fungizone) under the conditions of 37°C and 5% carbon dioxide. As a result, small free cells (ratio of longest diameter to shortest diameter: about 1 to 1.5) appeared within 48 hours (FIG. 2), and the number thereof increased with the lapse of time. The neuron differentiation medium containing the resulting small free cells was centrifuged to obtain a cell population. It was confirmed that the obtained cell population contains cells expressing nestin, $\beta$III-tubulin, Nanog, Oct 3/4, and SOX2.
The average diameter of the small free cells was measured with an optical microscope and found to be from

12 to 16 $\mu$m.

On the other hand, the average diameter of the cells adhered to the culture container was 20 $\mu$m or more, and the axons were elongated.

(3) Spheroid formation

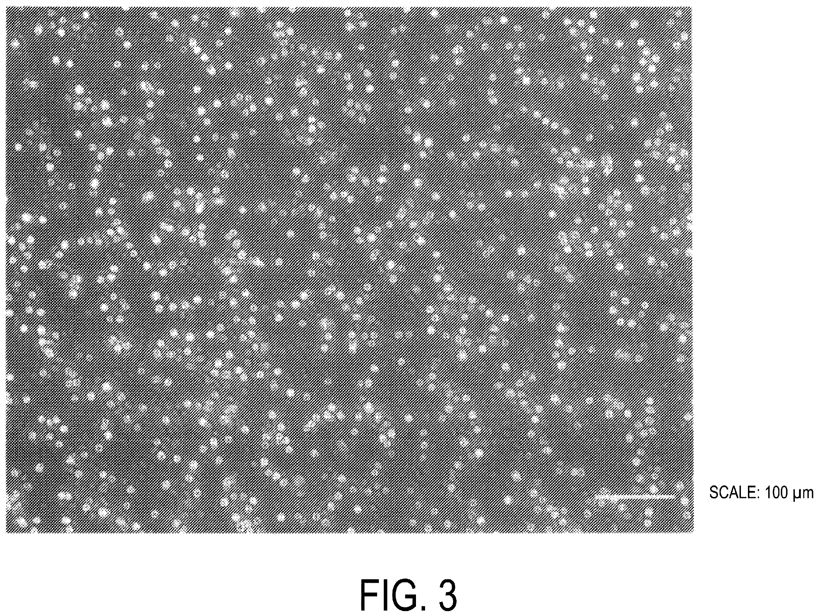

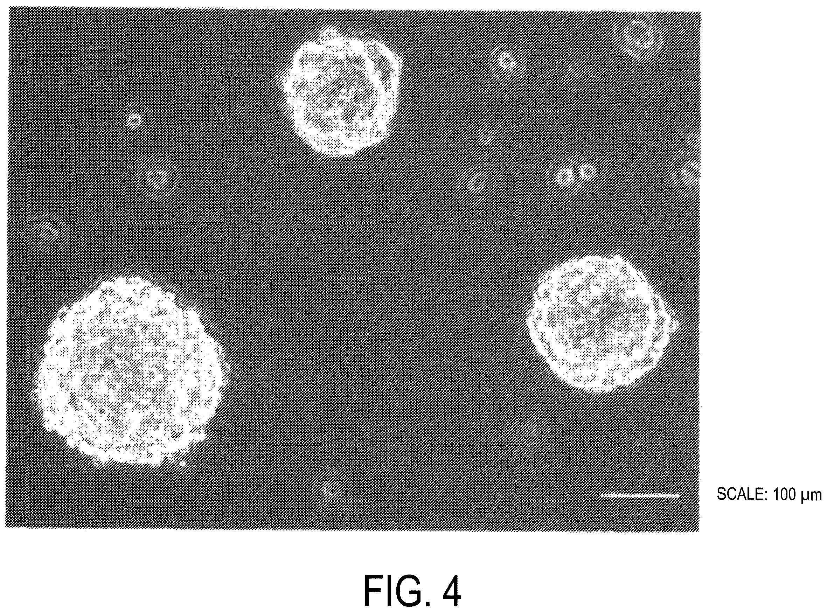

The cell population of free cells ($5 \times 10^5$ cells/60 mm culture dish) obtained in (2) was suspension-cultured in a non-adherent culture dish (product name: 3261 Corning (trade name), 60 mm ultra-low adhesion surface culture dish) using a neural maintenance medium (N-GRO, DV Biologics) (FIG. 3). As a result, about 80% of free cells formed spheroids after 24 hours (FIG. 4).

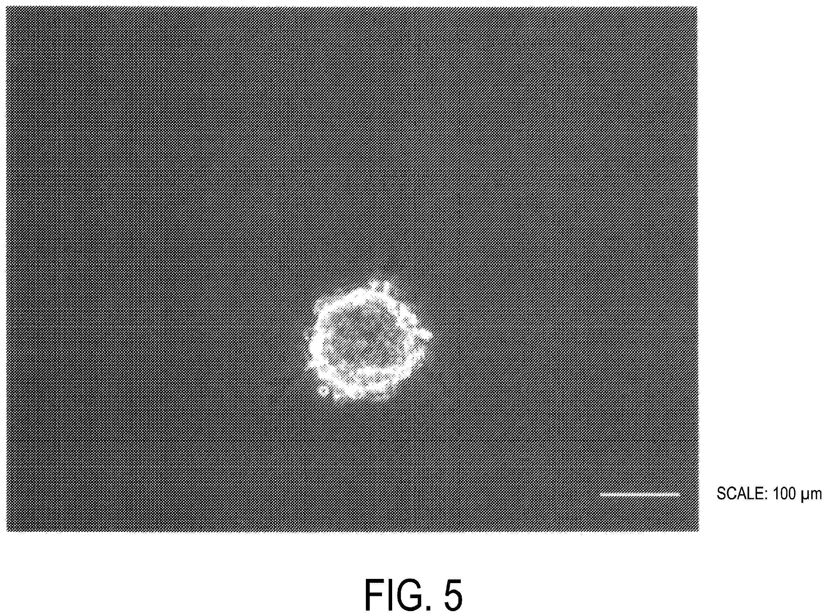

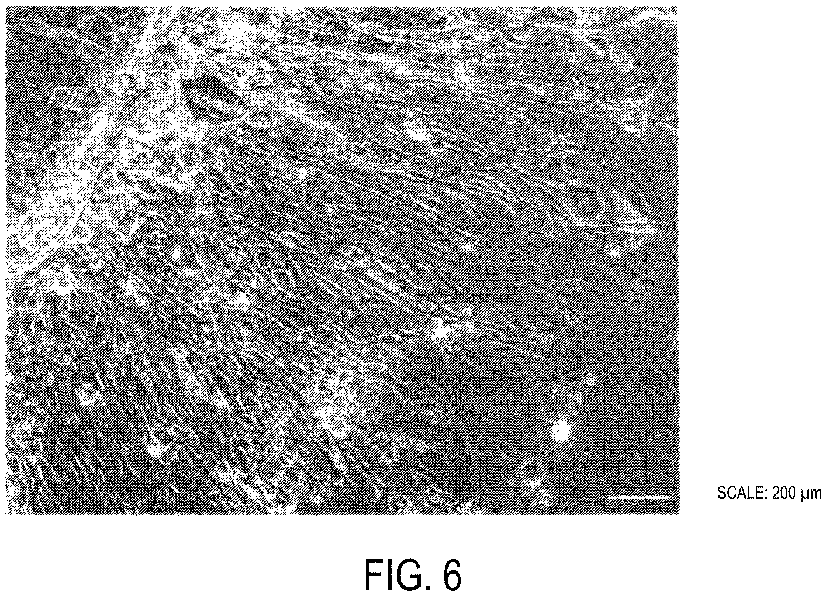

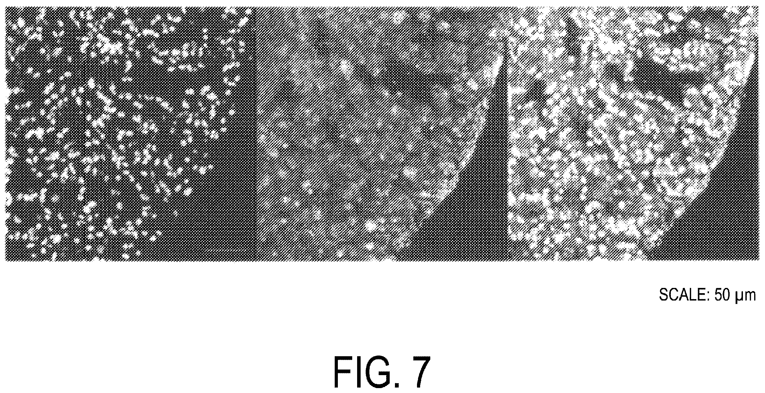

Further culture of the spheroids in the adherent culture dish containing the neural maintenance medium showed growth of neuron-like cells from the spheroids (FIG. 5 and FIG. 6). As a result of immunostaining for the spheroid, the spheroids were positive for staining with an anti-$\beta$III-tubulin antibody (FIG. 7). Here, $\beta$III-tubulin is a marker of neuronal microtubule elements.

(4) Confirmation of promotion of differentiation/differentiation into neural cells

After collection, the spheroids were dispersed using a digestive solution (0.25% trypsin, 0.02% EDTA/PBS (-)) into single cells and then cultured in Matrigel (trade name)-coated 60 mm culture dish for 96 hours under conditions of 37°C and 5% carbon dioxide in a neural maintenance medium. Then, the obtained cells were diluted at a dilution ratio of 1 : 3 and subcultured.

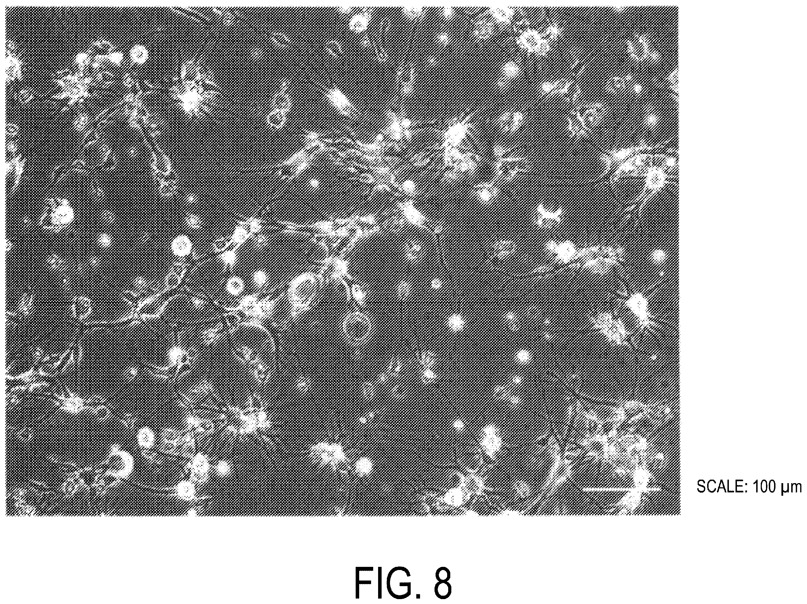

The spheroid-derived cells obtained by the subculture described above were rapidly differentiated into neural cells, and the generation of apolar nerval cells, unipolar nerval cells, bipolar nerval cells, and multipolar neural cells was observed (FIG. 8).

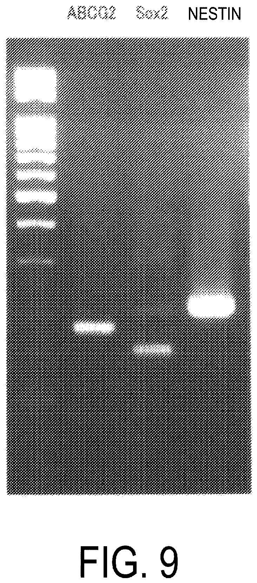

In addition, from the result of genetic analysis on the small free cells, it was confirmed that ABCG2, SOX2, and nestin were expressed (FIG. 9).

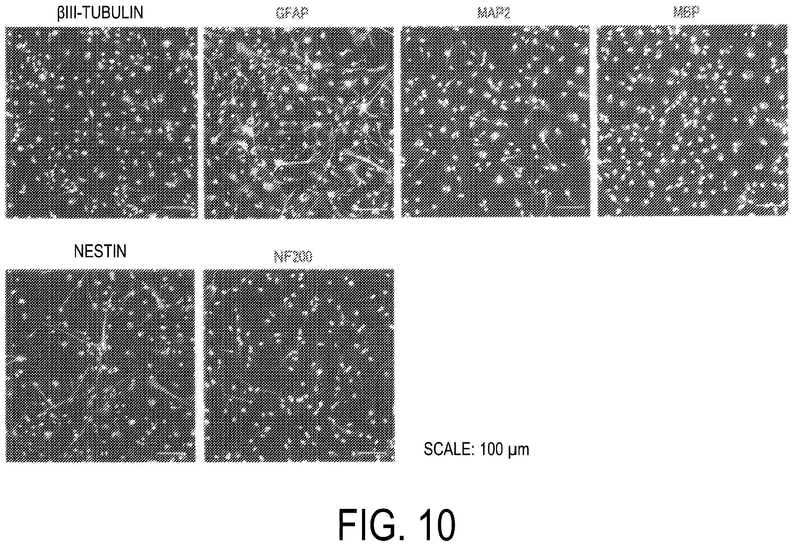

In addition, from the result of immunostaining on the small free cells, it was confirmed to be positive for nestin (a marker of immature neural stem cells in neurogenesis of the central nervous system), $\beta$III-tubulin (a marker of neuronal microtubule elements), NF200 (a marker of large myelinated neurons), MAP2 (a marker of intermediate filaments and microtubes), MBP (a marker of glial cells), and GFAP (a marker of intermediate filament proteins of astrocyte) (FIG. 10). From these results, it is considered that the cells obtained by subculture include neural stem cells, and immature or mature neural cells differentiated from the neural stem cells. In particular, since MBP positive was confirmed, it was confirmed that neural stem cells can be differentiated into glial cells.

From the results of (1) to (4), it was confirmed that the cell population obtained by collecting small free cells included neural stem cells.

Also, about 80% of the cells in the cell population form a spheroid in a short period of time of about 24 hours. Here, self-assembly to form a spheroid is a characteristic of neural cells including neural stem cells. Therefore, since the spheroid is rapidly formed, it is found that the cell population contains neural cells at high levels.

Test Example 2: Test for confirming normal diploidy of neural cells derived from dental pulp tissue

Preparation of test samples

According to the method described in (1) to (4) of Test Example 1 except that the primarily cultured cells were immediately cultured in the neuron differentiation medium without performing the culture described in (1) above after obtaining the cells from human dental pulp tissue, a neural cell population derived from the human dental pulp tissue was obtained and used as a test sample.

Preparation of reference sample

Cells from human dental pulp tissue were seeded in a neuron differentiation medium at $1 \times 10^4$ cells per 60 mm culture dish, and cultured at 37°C, 5% carbon dioxide for about 2 weeks. Here, the neuron differentiation medium consists of DMEM/F12 supplemented with 5% fetal bovine serum (FBS), 10 $\mu$M non-essential amino acids (NEAAs), 2 mM glutaMAX, 10 nM all-trans retinoic acid (ATRA), 50 $\mu$M ascorbic acid, 5 $\mu$M insulin, 10 nM dexamethasone, 20 nM progesterone, 20 nM estradiol, 10 nM nerve growth factor-1 (NGF-1), 10 ng/ml thyroxine (T4), 50 U/ml penicillin, 50 pg/ml streptomycin, and 0.25 pg/ml fungizone.

After the form of the cultured cells derived from human dental pulp tissue differentiated into neuron-like cells (about 70% cells), the medium was changed to a neural maintenance medium (N-GRO, DV Biologics). Neural cell colonies were isolated using small filter paper pieces. The isolated differentiated cells were cultured in the neural maintenance medium for about 2 to 4 weeks, and used as a reference sample in Test Example 2. In the subculture of the differentiated cells, precoating of a culture dish with Matrigel (trade name) (available from BD Falcon) was performed.

Preparation of chromosome distribution specimen

In accordance with the following procedure, for the test sample and the reference sample in Test Example 2, a chromosome distribution specimen was prepared using inducing-differentiated neural cells that reached confluence (P0: no subculture), and the chromosome number was measured.

1) In a 60 mm petri dish, $2 \times 10^4$ cells (or $1 \times 10^4$ to $4 \times 10^4$ cells/mL) were seeded in 3 mL DMEM/F12 (10% FBS) and cultured in a $CO_2$ incubator.
2) After 3 days (or after 3 to 5 days), after cell proliferation was confirmed, Colcemid (Sigma) was added to the culture solution at $10^{-7}$ M, and the petri dish was placed in a $CO_2$ incubator.
3) After 4 to 6 hours, the culture solution was quietly removed, 0.25% trypsin was added, and the mixture was warmed for 10 minutes.
4) The floating cells in the petri dish were placed in a 50 mL centrifuge tube and centrifuged at $100 \times$ g for 5 minutes.
5) 5 mL of 0.075 M KCl aqueous solution (36.5°C) was placed in a centrifuge tube to resuspend the cells, and left at room temperature for 20 minutes.
6) To the centrifuge tube was added 5 mL of an acidic methanol solution (acetic methanol, available from Nacalai Tesque, Inc.) cooled to 0°C, and the mixture was lightly mixed. Thereafter, the mixture was centrifuged at $100 \times$ g for 2 minutes.
7) A supernatant in the centrifuge tube was discarded, a residue was added to a vortex mixer, an acidic methanol solution was quietly added, and the mixture was quietly stirred.
8) The centrifuge tube was left on ice for 10 minutes.
9) The centrifuge tube was centrifuged at $100 \times$ g for 2 minutes.
10) The supernatant in the centrifuge tube was discarded, 0.2 mL of the acidic methanol solution was added, and the centrifuge tube was vibrated to suspend the cells.
11) The cell suspension was collected from the centrifuge tube with a pipette, and one drop was added to a glass slide on ice.
12) The glass slide was dried over boiling water previously placed in a beaker.
13) It was confirmed using a phase contrast microscope that the chromosomes of the glass slide cells spread.
14) Giemsa staining of cells was performed under a microscope, and the cells were washed with water.
15) The glass slide was air-dried, and the number of chromosomes was counted for those in which one cell was confirmed under a microscope (objective lens: 40 to 100 times).

In the preparation of the chromosome distribution specimen in Test Example 2, test samples were obtained from three persons, and reference samples were obtained from five persons. In addition, chromosome distribution specimens were prepared for 100 cells for each human sample, and the proportion of normal chromosomes was calculated. The results are shown in Table 3.

[Table 3]

Table 3

| Type | Sample | | | | Number of chromosomes | | | | | | | | | | | | | | | Proportion of normal diploid cells (%) | Average percentage of normal diploid cells per sample type (%) |
|---|---|---|---|---|---|---|---|---|---|---|---|---|---|---|---|---|---|---|---|---|---|
| | Source tissue | Subject | | Preparation per sample | 40 | 41 | 42 | 43 | 44 | 45 | 46 (Nor) | 47 | 48 | 49 | 50 | 51 | 52 | 53 | 54 | | |
| | | Age (years) | Gender | | | | | | | | | | | | | | | | | | |
| Test sample | D | 25 | | 7 | 0 | 0 | 0 | 0 | 0 | 0 | 100 | 0 | 0 | 0 | 0 | 0 | 0 | 0 | 0 | 100 | 99.7 |
| Test sample | D | 17 | | 8 | 0 | 0 | 0 | 0 | 0 | 0 | 100 | 0 | 0 | 0 | 0 | 0 | 0 | 0 | 0 | 100 | |
| Test sample | D | 30 | | 7 | 0 | 0 | 0 | 0 | 0 | 0 | 99 | 1 | 0 | 0 | 0 | 0 | 0 | 0 | 0 | 99 | |
| Reference sample | D | 16 | | 46 | 0 | 2 | 0 | 0 | 2 | 3 | 74 | 6 | 4 | 2 | 1 | 2 | 2 | 1 | 0 | 74 | 79.2 |
| Reference sample | D | 30 | | 52 | 0 | 0 | 0 | 2 | 2 | 2 | 80 | 4 | 3 | 2 | 2 | 2 | 1 | 1 | 0 | 80 | |
| Reference sample | D | 25 | | 45 | 0 | 1 | 0 | 0 | 2 | 2 | 79 | 7 | 4 | 2 | 2 | 1 | 1 | 1 | 0 | 79 | |
| Reference sample | D | 22 | | 57 | 1 | 0 | 0 | 0 | 2 | 2 | 81 | 6 | 3 | 2 | 0 | 0 | 0 | 0 | 0 | 81 | |
| Reference sample | D | 17 | | 56 | 0 | 0 | 0 | 0 | 2 | 2 | 82 | 10 | 2 | 2 | 0 | 0 | 0 | 0 | 0 | 82 | |

In Test Example 2, the average percentage of normal diploid cells in the test samples was about 100%. On the other hand, the average percentage of the normal diploid cells in the reference sample was about 79%. From this result, it was confirmed that the occurrence of chromosomal abnormalities was suppressed at high levels in the test

16

sample derived from the dental pulp tissue.

Test Example 3: Test for confirming normal diploidy of neural cells derived from gingival tissue

According to the method described in Test Example 2 except that the cells were obtained from the human gingival tissue (dental pulp tissue was replaced with gingival tissue), a confirmation test for the normal diploidy of the neural cells derived from the human gingival tissue was performed. In the preparation of the chromosome distribution specimen in Test Example 3, test samples were obtained from three persons, and reference samples were obtained from five persons. The results are shown in Table 4.

[Table 4]

Table 4

| Type | Sample | | | Preparation period of sample cells (days) | Number of chromosomes | | | | | | | | | | | | | | | | Proportion of normal diploid cells (%) | Average percentage of normal diploid cells per sample type (%) |
|---|---|---|---|---|---|---|---|---|---|---|---|---|---|---|---|---|---|---|---|---|---|---|---|
| | Source tissue | Subject | | | 40 | 41 | 42 | 43 | 44 | 45 | 46 (Normal diploid) | 47 | 48 | 49 | 50 | 51 | 52 | 53 | 54 | | |
| | | Age (years) | Gender | | | | | | | | | | | | | | | | | | | |
| Test sample | Gingiva | 22 | Female | 8 | 0 | 0 | 0 | 0 | 0 | 1 | 98 | 1 | 0 | 0 | 0 | 0 | 0 | 0 | 0 | 98 | 99.0 |
| Test sample | Gingiva | 43 | Male | 9 | 1 | 0 | 0 | 0 | 0 | 0 | 100 | 0 | 0 | 0 | 0 | 0 | 0 | 0 | 0 | 100 | |
| Reference sample | Gingiva | 31 | Female | 47 | 0 | 0 | 2 | 2 | 0 | 2 | 78 | 5 | 3 | 2 | 0 | 2 | 2 | 0 | 1 | 78 | 82.0 |
| Reference sample | Gingiva | 25 | Female | 52 | 0 | 0 | 2 | 2 | 2 | 2 | 82 | 4 | 3 | 2 | 2 | 0 | 1 | 0 | 0 | 82 | |
| Reference sample | Gingiva | 19 | Female | 45 | 1 | 1 | 1 | 0 | 0 | 3 | 86 | 3 | 2 | 2 | 2 | 1 | 0 | 2 | 2 | 85 | |

In Test Example 3, the average percentage of normal diploid cells in the test samples was about 99%. On the other hand, the average percentage of the normal diploid cells in the reference sample was about 82%. From this result, it was confirmed that the occurrence of chromosomal abnormalities was suppressed at high levels in the test sample derived from the gingival tissue.

Test Example 4: Test for confirming normal diploidy of neural cells derived from buccal fat pad tissue

According to the method described in Test Example 2 except that the cells were obtained from the human buccal fat pad tissue (dental pulp tissue was replaced with buccal fat pad tissue), a confirmation test for the normal diploidy of the neural cells derived from the human buccal fat pad tissue was performed. In the preparation of the chromosome distribution specimen in Test Example 4, test samples were obtained from one person, and reference samples were obtained from two persons. The results are shown in Table 5.

[Table 5]

Table 5

| Sample | | | | Preparation period of sample cells (days) | Number of chromosomes | | | | | | | | | | | | | | | Proportion of normal diploid cells (%) | Average percentage of normal diploid cells per sample type (%) |
| --- | --- | --- | --- | --- | --- | --- | --- | --- | --- | --- | --- | --- | --- | --- | --- | --- | --- | --- | --- | --- | --- |
| Type | Subject | | Source tissue | | 40 | 41 | 42 | 43 | 44 | 45 | 46 (Normal diploid) | 47 | 48 | 49 | 50 | 51 | 52 | 53 | 54 | | |
| | Age (years) | Gender | | | | | | | | | | | | | | | | | | | |
| Test sample | 21 | Male | Buccal fat pad | 8 | 0 | 0 | 0 | 0 | 0 | 2 | 97 | 1 | 0 | 0 | 0 | 0 | 0 | 0 | 0 | 98 | 98.0 |
| Reference sample | 20 | Male | Buccal fat pad | 51 | 0 | 2 | 2 | 3 | 0 | 6 | 78 | 5 | 3 | 0 | 2 | 0 | 1 | 0 | 0 | 78 | 75.0 |
| Reference sample | 18 | Female | Buccal fat pad | 61 | 1 | 2 | 0 | 0 | 1 | 5 | 72 | 8 | 4 | 3 | 2 | 2 | 1 | 1 | 1 | 72 | |

In Test Example 4, the average percentage of normal diploid cells in the test samples was about 98%. On the other hand, the average percentage of the normal diploid cells in the reference sample was about 75%. From this result, it was confirmed that the occurrence of chromosomal abnormalities was suppressed at high levels in the test sample derived from the buccal fat pad tissue.

Reference Test Example 1: Test for confirming normal diploidy of neural cells derived from bone marrow tissue

Cells were obtained from bone marrow tissue, and only a reference sample (without adjustment of a test sample) was prepared according to the method described in Test Example 2 (human dental pulp tissue was replaced with bone marrow tissue), and a confirmation test for normal diploidy of neural cells was performed. Note that, in the preparation of the chromosome distribution specimen in Reference Test Example 1, the reference sample was obtained from three persons. The results are shown in Table 6.

[Table 6]

Table 6

| Sample | | | | Preparation period of sample cells (days) | Number of chromosomes | | | | | | | | | | | | | | | Proportion of normal diploid cells (%) | Average percentage of normal diploid cells per sample (%) |
|---|---|---|---|---|---|---|---|---|---|---|---|---|---|---|---|---|---|---|---|---|---|---|
| Type | Source tissue | Subject | | | 40 | 41 | 42 | 43 | 44 | 45 | 46 (Normal diploid) | 47 | 48 | 49 | 50 | 51 | 52 | 53 | 54 | | |
| | | Age (years) | Gender | | | | | | | | | | | | | | | | | | |
| Reference sample | Bone marrow | 31 | Male | 51 | 0 | 0 | 0 | 1 | 3 | 5 | 81 | 4 | 1 | 2 | 0 | 2 | 1 | 0 | 0 | 81 | 78.7 |
| Reference sample | Bone marrow | 44 | Male | 44 | 1 | 0 | 0 | 1 | 2 | 2 | 79 | 8 | 2 | 2 | 1 | 2 | 0 | 0 | 0 | 79 | |
| Reference sample | Bone marrow | 37 | Male | 50 | 0 | 0 | 1 | 1 | 0 | 2 | 76 | 8 | 4 | 2 | 2 | 2 | 0 | 1 | 1 | 76 | |

In Reference Test Example 1, the average percentage of the normal diploid cells in the reference sample was 78.7%, which was less than 80%, suggesting that about 20% of chromosomal abnormalities occurred.

Reference Test Example 2: Test for confirming normal diploidy of neural cells derived from subcutaneous adipose tissue

Cells were obtained from subcutaneous adipose tissue, and only a reference sample (without adjustment of a test sample) was prepared according to the method described in Test Example 2 (human dental pulp tissue was replaced with subcutaneous adipose tissue), and a confirmation test for normal diploidy of neural cells was performed. Note that, in the preparation of the chromosome distribution specimen in Reference Test Example 2, the reference sample was obtained from two persons. The results are shown in Table 7.

[Table 7]

Table 7

| | Sample | | | | | Number of chromosomes | | | | | | | | | | | | | | | | Proportion of normal diploid cells (%) | Average percentage of normal diploid cells per sample type (%) |
|---|---|---|---|---|---|---|---|---|---|---|---|---|---|---|---|---|---|---|---|---|---|---|---|
| | | Subject | | Preparation period of sample cells (days) | | 40 | 41 | 42 | 43 | 44 | 45 | 46 (Normal diploid) | 47 | 48 | 49 | 50 | 51 | 52 | 53 | 54 | | |
| Type | Source tissue | Age (years) | Gender | | | | | | | | | | | | | | | | | | | |
| Reference sample | Subcutaneous adipose | 40 | Female | 48 | | 1 | 1 | 1 | 0 | 1 | 2 | 78 | 7 | 4 | 1 | 2 | 1 | 0 | 1 | 0 | 78 | 76.0 |
| Reference sample | Subcutaneous adipose | 38 | Female | 49 | | 0 | 0 | 1 | 1 | 1 | 2 | 74 | 6 | 5 | 2 | 2 | 3 | 0 | 1 | 2 | 74 | |

In Reference Test Example 2, the average percentage of the normal diploid cells in the reference sample was 76.0%, which was less than 80%, suggesting that about 20% of chromosomal abnormalities occurred.

Sequence Listing

232202PXseq.txt

**Claims**

1. A method for producing a neural cell-containing preparation, the method comprising:

   preparing an adherent cell culture container in which initially cultured cells derived from intraoral mesenchymal tissue are cultured in a neuron differentiation medium; and
   collecting cells that do not adhere to the adherent cell culture container from the adherent cell culture container to obtain a cell population,
   wherein the neural cell-containing preparation includes the cell population, or spheroids or neural cells derived from the cell population.

2. The method according to claim 1, wherein a period from start of the initial culture to acquisition of the neural cell-containing preparation is from 2 to 10 days.

3. The method according to claim 1 or 2, wherein the cells that do not adhere to the adherent cell culture container are free cells present in the neuron differentiation medium or cells further adhering to cells that adhere to the adherent cell culture container.

4. The method according to any one of claims 1 to 3, wherein the intraoral mesenchymal tissue is selected from the group consisting of gingival tissue, submucous tissue of cheek, buccal fat pad tissue, and dental pulp tissue.

5. The method according to any one of claims 1 to 4, wherein the intraoral mesenchymal tissue is human tissue.

6. The method according to any one of claims 1 to 5, wherein the initially cultured cells are primarily cultured cells or cells subcultured for 2 to 5 passages.

7. The method according to any one of claims 1 to 6, wherein the cells that do not adhere to the adherent cell culture container have an average diameter from 12 to 16 $\mu$m.

8. The method according to any one of claims 1 to 7, wherein a period during which the initially cultured cells are cultured in the neuron differentiation medium is from 0.5 to 10 days.

9. The method according to any one of claims 1 to 8, wherein a cell-contacting surface of the container containing the neuron differentiation medium is adhesive to neural cells of which axons are elongated.

10. The method according to any one of claims 1 to 9, wherein collection of the free cells is performed by centrifugation, aspiration, a magnetic bead method, or a FACS method.

11. The method according to any one of claims 1 to 10, further comprising:
culturing the cell population to form spheroids.

12. The method according to claim 11, wherein a period for culturing the cell population in the spheroid formation is from 0.5 to 2 days.

13. The method according to claim 11 or 12, wherein a cell-contacting surface of a culture container used for culturing the cell population in the spheroid formation is non-adherent to neural cells of which axons are elongated.

14. The method according to any one of claims 11 to 13, wherein the culture of the cell population in the spheroid formation is performed by suspension culture, stirring culture, rotary culture, or shaking culture.

15. The method according to any one of claims 11 to 14, further comprising:
dispersing and culturing the spheroids.

16. The method according to claim 15, wherein a period for culturing cells obtained by dispersing the spheroids is from 3 hours to 7 days.

17. The method according to any one of claims 1 to 16, wherein the neural cell-containing preparation contains neural stem cells.

18. The method according to any one of claims 1 to 17, wherein the neural cell-containing preparation is a preparation for regenerative medicine.

19. The method according to any one of claims 1 to 18, wherein the neural cell-containing preparation is a preparation for autologous transplantation or allogenic transplantation.

20. The method according to any one of claims 1 to 19, wherein the neural cell-containing preparation is used for repairing nervous tissue damage.

21. A neural cell-containing preparation obtained by the method described in any one of claims 1 to 20.

22. A neural cell population derived from intraoral mesenchymal tissue cells, wherein a proportion of normal diploid cells is 80% or more.

23. The neural cell population according to claim 22, wherein the proportion of the normal diploid cells is 90% or more.

24. The neural cell population according to claim 22 or 23, wherein the intraoral mesenchymal tissue cells are primarily cultured cells or cells subcultured for 2 to 5 passages.

25. The neural cell population according to any one of claims 22 to 24, wherein the intraoral mesenchymal tissue is selected from the group consisting of gingival tissue, submucous tissue of cheek, buccal fat pad tissue, and dental pulp tissue.

26. The neural cell population according to any one of claims 22 to 25, wherein the intraoral mesenchymal tissue is human tissue.

27. The neural cell population according to any one of claims 22 to 26, which is obtained by the method according to claim 15.

28. A pharmaceutical preparation comprising the neural cell population according to any one of claims 22 to 27.

29. The pharmaceutical preparation according to claim 28, which is a preparation for regenerative medicine.

30. The pharmaceutical preparation according to claim 28 or 29, which is a preparation for autologous transplantation or allogenic transplantation.

31. The pharmaceutical preparation according to claim 30, which is used for repairing nervous tissue damage.

SCALE: 100 µm

FIG. 1

SCALE: 100 μm

FIG. 2

SCALE: 100 μm

FIG. 3

SCALE: 100 μm

FIG. 4

SCALE: 100 μm

FIG. 5

SCALE: 200 µm

FIG. 6

SCALE: 50 μm

FIG. 7

SCALE: 100 μm

FIG. 8

ABCG2    Sox2    NESTIN

FIG. 9

βIII-TUBULIN  GFAP  MAP2  MBP

NESTIN  NF200

SCALE: 100 μm

FIG. 10

<div align="center">**INTERNATIONAL SEARCH REPORT**</div>

| International application No. |
| --- |
| PCT/JP2020/019290 |

**A. CLASSIFICATION OF SUBJECT MATTER**
A61P 25/00(2006.01)i; C12N 5/079(2010.01)i; C12N 5/0797(2010.01)i; A61K 35/30(2015.01)i; A61K 35/38(2015.01)i; A61L 27/38(2006.01)i
FI: A61K35/38; C12N5/079; C12N5/0797; A61P25/00; A61L27/38 300; A61K35/30
According to International Patent Classification (IPC) or to both national classification and IPC

**B. FIELDS SEARCHED**

Minimum documentation searched (classification system followed by classification symbols)
A61P25/00; C12N5/079; C12N5/0797; A61K35/30; A61K35/38; A61L27/38

| Documentation searched other than minimum documentation to the extent that such documents are included in the fields searched | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | JP 2016-517405 A (AVITA INTERNATIONAL LTD.) 16.06.2016 (2016-06-16) in particular, claims 1, 97, example 18, fig. 76G | 21-31<br>25-31<br>1-20 |
| X<br>Y<br>A | TAMAKI, Y. et al., "In vitro analysis of mesenchymal stem cells derived from human teeth and bone marrow.", Odontology, 2013, vol. 101, pp. 121-132, DOI: 10.1007/s10266-012-0075-0, in particular, abstract, fig. 1, p. 128, fig. 4, 5, p. 132, right column, the last paragraph | 21-31<br>25-31<br>1-20 |
| Y | FOURNIER, B. P. et al., "CHARACTERISATION OF HUMAN GINGIVAL NEURAL CREST – DERIVED STEM CELLS IN MONOLAYER AND NEUROSPHERE CULTURES", European Cells and Materials., 2016, vol. 31, pp. 40-58, DOI: 10.22203/eCM.v031a04, in particular, abstract | 25-31 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 July 2020 (17.07.2020) | 28 July 2020 (28.07.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**

| | International application No. |
|---|---|
| | PCT/JP2020/019290 |

C (Continuation).   DOCUMENTS CONSIDERED TO BE RELEVANT

| Category* | Citation of document, with indication, where appropriate, of the relevant passages | Relevant to claim No. |
|---|---|---|
| Y | 高橋 悠 ほか，ヒト頬脂肪体由来幹細胞を細胞源とした神経再生療法に関する検討, J Oral Biosci Suppl., vol. 57, 2015, p. 299(P1-55), ISSN : 2187-9109, entirety, non-official translation (TAKAHASHI, Haruka et al., "Study on nerve regeneration therapy using human buccal fat pad-derived stem cells as a cell source") | 25-31 |
| A | XIAO, L. et al., "Human dental pulp cells differentiate toward neuronal cells and promote neuroregeneration in adult organotypic hippocampal slices in vitro.", Int J Mol Sci., 2017, vol. 18, Article No. 1745(pp. 1-13), doi: 10.3390/ijms18081745, in particular, abstract | 1-20 |
| A | WO 2018/190305 A1 (AGC INC.) 18.10.2018 (2018-10-18) in particular, claims, etc. | 1-20 |
| A | WO 2009/148170 A1 (INST OF PHYSICAL & CHEMICAL RES) 10.12.2009 (2009-12-10) in particular, claims, etc. | 1-20 |

Form PCT/ISA/210 (continuation of second sheet) (January 2015)

**INTERNATIONAL SEARCH REPORT**
Information on patent family members

| International application No. |
| --- |
| PCT/JP2020/019290 |

| Patent Documents referred in the Report | Publication Date | Patent Family | Publication Date |
| --- | --- | --- | --- |
| JP 2016-517405 A | 16 Jun. 2016 | WO 2014/141210 A2 claims 1, 97, example 18, fig. 76G US 2015/0050248 A1 EP 2970880 A2 KR 10-2018-0137488 A CN 109219441 A | |
| WO 2018/190305 A1 | 18 Oct. 2018 | (Family: none) | |
| WO 2009/148170 A1 | 10 Dec. 2009 | US 2011/0091869 A1 claims EP 2314671 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019091625 A

- JP 2010060576 A

**Non-patent literature cited in the description**

- **OKANO H ; OKADA S ; NAKAMURA M ; TOYAMA Y.** Neural stem cells and regeneration of injured spinal cord. *Kidney International,* 2005, vol. 68, 1927-31
- **KAUKUA N ; SHAHIDI MK ; KONSTANTINIDOU C ; DYACHUK, V ; KAUCKA M ; FURLAN A ; AN Z ; WANG L ; HULTMAN I ; AHRLUND-RIVHTER L.** Glial origin of mesenchymal stem cells in a tooth model system. *Nature,* 25 September 2014, vol. 513 (7519), 551-4
- **JANG S ; JEONG HS.** *Histone deacetylase inhibition-mediated neuronal differentiation via the Wnt signaling pathway in human adipose tissue derived mesenchymal stem cells*

- **DONG F ; FEND E ; ZHENG T ; TIAN Y.** situ synthesized silver nanoclusters for tracking the role of telomerase activity in the differentiation of mesenchymal stem cells top neural stem cells. *ACS Appl Mate Interfaces,* 17 January 2018, vol. 10 (2), 2051-2057
- **LI XIAO ; RYOJI IDE ; CHIKAKO SAIKI ; YASUO KUMAZAWA ; HISASHI OKAMURA.** Human Dental Pulp Cells Differentiate toward Neuronal Cells and Promote Neuroregeneration in Adult Organotypic Hippocampal Slices In Vitro. *Int. J. Mol. Sci.,* 2017, vol. 18, 1745